# EUROPEAN PATENT SPECIFICATION

(11) **EP 2 195 456 B1**
(45) Date of publication and mention of the grant of the patent: **28.11.2012**
(21) Application number: 08802770.1
(22) Date of filing: 02.10.2008
(51) Int. Cl.: C12Q 1/68

(54) **USE OF POLYMORPHISMS IN THE TMEM132D GENE IN THE PREDICTION AND TREATMENT OF ANXIETY DISORDERS**
VERWENDUNG VON POLYMORPHISMEN IM TMEM132D-GEN BEI DER VORHERSAGE UND BEHANDLUNG VON ANGSTZUSTÄNDEN
UTILISATION DE POLYMORPHISMES DU GÈNE TMEM132D DANS LA PRÉDICTION ET LE TRAITEMENT DE TROUBLES DE L'ANXIÉTÉ

(30) Priority: 05.10.2007 US 977780 P
(43) Date of publication of application: 16.06.2010
(73) Proprietor: Max-Planck-Gesellschaft zur Förderung der Wissenschaften e.V., 80539 München (DE)
(72) Inventor: MÜLLER-MYHSOK, Bertram, 81241 Munich (DE); ERHARDT, Angelika, 81927 Munich (DE); UHR, Manfred, 82131 Stockdorf (DE); BETTECKEN, Thomas, 80939 Munich (DE); HOLSBOER, Florian, 80805 Munich (DE)
(74) Representative: Weiss, Wolfgang
(86) International application number: PCT/EP2008/008382
(87) International publication number: WO 2009/043584

(56) References cited:
- WO-A-00/05407
- WO-A-2005/040427
- US-A1- 2002 160 390
- US-A1- 2006 024 715
- MARON ET AL: "Association study of tryptophan hydroxylase 2 gene polymorphisms in panic disorder" NEUROSCIENCE LETTERS, LIMERICK, IE, vol. 411, no. 3, 2 December 2006 (2006-12-02), pages 180-184, XP005792039 ISSN: 0304-3940
- THOERINGER ET AL: "Association of a Met88Val diazepam binding inhibitor (DBI) gene polymorphism and anxiety disorders with panic attacks" JOURNAL OF PSYCHIATRIC RESEARCH, ELSEVIER LTD, GB, vol. 41, no. 7, 7 March 2007 (2007-03-07), pages 579-584, XP005913169 ISSN: 0022-3956
- ERHARDT ET AL: "Association of polymorphisms in P2RX7 and CaMKKb with anxiety disorders" JOURNAL OF AFFECTIVE DISORDERS, ELSEVIER BIOCHEMICAL PRESS, AMSTERDAM, NL, vol. 101, no. 1-3, 9 June 2007 (2007-06-09), pages 159-168, XP022109939 ISSN: 0165-0327

## Description

The present invention relates to methods, compositions, kits and reagents for the diagnosis and treatment of anxiety disorders.

Panic disorder (PD) is characterized by sudden episodes of acute anxiety or intense fear that may occur without any apparent reason or stimulus. PD is often accompanied by agoraphobia, an avoidance of situations where a person may feel trapped and unable to escape. The life-time prevalence of PD is up to 4% worldwide and there is substantial evidence that genetic factors contribute to the development of panic disorder (Kessler et al., 2005). Twin, family, and segregation analysis studies indicate a definite but complex genetic basis in PD with a potential involvement of multiple genes, each of modest effect. The estimates for genetic heritability in PD ranges up to 30-40% and in agoraphobia up to 50% (Kendler 2001, Hettema et al., 2001). In the past, genetic studies were mainly designed as candidate gene studies or linkage studies. In linkage studies, DNA markers across the genome are genotyped in large pedigrees or affected relative pairs to determine the chromosomal location of susceptibility genes (for review: Fyer et al., 2006). Using the candidate gene approach, promising genes or gene systems were investigated in anxiety and panic disorder, but, only a few promising genes have been studied so far and a low number of associations have been identified despite numerous studies and enormous effort. Furthermore the replication of these findings often failed in independent studies, facing the problems of phenotypic diversity, genetic heterogeneity and differences in the sample sizes.

Maron et al., 2007 (Neuroscience letters) discloses an association between an SNP (rs1386494) located in the TPH2 gene and female patients with panic disorder, indicating a possible gender-specific effect of TPH2 gene variants in panic disorder.

In summary, recent genome scans for PD using candidate genes or linkage approach have produced a number of significant peaks and associations but, failed to bring up definitive results or answers concerning the genetic basis of panic disorder. Until now no polymorphisms have been identified which have a clear and significant association with panic and anxiety disorders. The identification of such polymorphisms may enable the development of new therapeutics with minimal side effects, diagnostics and/or methods for screening for modulators of panic and anxiety disorders.

Following the development of high-throughput chip-based genotyping technologies, cost-effective simultaneous genotyping of many SNPs across the genome became possible. The Genome-wide association (GWA) scans represent a new exciting opportunity to identify common genetic variants that predispose to human psychiatric disease without the restriction to candidate genes or requirement of strong prior hypothesis (NIC NHGRI working Group, 2007).

In the studies on which the present application is based the inventors first performed a genome-wide case-control association analysis in patients with PD and PDA. To optimize the power and the cost-effectiveness, the study was performed in a two-stage design by excluding markers with little evidence of association in the first stage for further testing (Skol et al., 2007). In the first stage, a panic sample consisted of 216 cases and 220 matched controls was individually genotyped using a 300k Illumina Bead Array Chip. In the second stage, SNPs with the best p-values were analysed in an independent German case-control sample from Münster. Positive association obtained in the second stage were verified in the joint sample, to decrease the possibility of false positive findings. The results suggest an implication of several genes in the risk for panic disorder. One association was most promising showing an increase of association in the joint sample. This first genome-wide association study in PD brought up promising results and has important implication for future genetic and biological studies in PD.

Surprisingly, the inventors detected an association of genetic variances in several genes with anxiety disorders. Polymorphisms in the TMEM132D, FHIT, KPNA4, CRIM1 and PLCB1 genes were found to be particularly relevant. More particular, polymorphisms in the TMEM132D gene were identified, which have a clear and statistically relevant association with anxiety disorders.

A first aspect of the invention relates to a method for the diagnosis of an anxiety disorder or a predisposition therefor in a mammal, particularly in a human being, comprising determining the presence of at least one polymorphism in the TMEM123D gene, wherein said at least one polymorphism is associated with said anxiety disorder or predisposition therefor.

Polymorphisms in the TMEM123D, FHIT, KPNA4, CRIM1 and PLCB1 genes have a statistically significant association is preferably p < 0.05, more preferably p< 0.01 and most preferably p < 0.001. The determination of significance may be carried out by a polymorphism/haplotype analysis of a sufficient number, e.g. of at least 40, preferably of at least 80, more preferably of at least 100 normal cases. More preferably the determination of significance may be carried out as described in the Example section.

Surprisingly, it was found that polymorphisms associated with anxiety disorders may particularly occur in introns of the TMEM132D, FHIT, KPNA4, CRIM1 and PLCB1 genes, in particular in the intron of the TMEM132D gene.

Examples for polymorphisms having a particularly clear and statistically relevant association with anxiety disorders are given in table 1.

**Table 1: SNPs selected for stage 2 genotyping regarding the best p-value in stage 1.**

| **SNP** | **Chr** | **Gene** | **p_nominal** | | **SNP** | **Chr** | **Gene** | **p_nominal** |
|---|---|---|---|---|---|---|---|---|
| **rs7309727** | 12 | TMEM132D | 5,43E-007 | | **rs4669018** | 2 | MYCN | 3,15E-006 |
| **rs10267387** | 7 | CREB5 | 8,99E-006 | | **rs739894** | 22 | OSBP2 | 2,73E-005 |
| **rs3761347** | 10 | SH3PXD2A | 9,27E-006 | | **rs7736147** | 5 | LOC375449 | 2,87E-005 |
| **rs6918969** | 6 | NFYA/C6orf130 | 2,88E-005 | | **rs4714421** | 6 | UNC5CL | 3,89E-005 |
| **rs203838** | 1 | SAC | 3,03E-005 | | **rs2059873** | 16 | SYT17 | 4,13E-005 |
| **rs2271339** | 4 | LPHN3 | 3,73E-005 | | **rs5766175** | 22 | PHF21B | 4,67E-005 |
| **rs2075520** | 16 | ZP2 | 4,64E-005 | | **rs10813843** | 9 | TAF1L | 5,14E-005 |
| **rs2035262** | 17 | FLJ42461 | 8,55E-005 | | **rs3753617** | 1 | RYR2 | 5,36E-005 |
| **rs2297518** | 17 | NOS2A | 9,65E-005 | | **rs6474855** | 9 | FREM1 | 5,82E-005 |
| **rs8118127** | 20 | PLCB1 | 9,86E-005 | | **rs9428379** | 1 | RYR2 | 0,000119 |
| **rs754799** | 19 | SCAMP4/LOC113179 | 9,81E-005 | | **rs471972** | 1 | DPT | 5,10E-006 |
| **rs12466450** | 2 | CRIM1 | 0,000461 | | **rs1805377** | 5 | XRCC4 | 6,59E-005 |
| **rs4740624** | 9 | C9orf93 | 3,04E-005 | | **rs6767155** | 3 | TSC22D2 | 4,89E-005 |
| **rs414273** | 18 | DLGAP1 | 4,33E-005 | | **rs11943595** | 4 | ELOVL6 | 8,93E-005 |
| **rs12629266** | 3 | ITGA9 | 4,92E-005 | | **rs4391483** | 9 | PTPRD | 0,000159 |
| **rs4522024** | 1 | COL24A1 | 6,59E-005 | | **rs2536090** | 7 | PRKAG2 | 2,25E-005 |
| **rs2734605** | 7 | CUTL1 | 7,23E-005 | | **rs1931170** | 3 | NAALADL2 | , 6,80E-005 |
| **rs3905267** | 4 | GALNT17 | 7,81E-005 | | **rs3748483** | 20 | SLC24A3 | 9,29E-05 |
| **rs1060369** | 12 | TMEM132D | 1,44E-005 | | **rs597520** | 12 | K6IRS3 | 6,43E-005 |
| **rs3746721** | 20 | SIRPG | 0,000167 | | **rs10486593** | 7 | CREB5 | 5,01E-005 |
| **rs3775801** | - 4 | OTUD4 - | 1,35E-005 | | **rs1348036** | 3 | intragenic | 8,54E-005 |
| **rs11060369** | 12 | TMEM132D | 1,44E-005 | | **rs4559963** | 17 | NF1 | 2,19E-005 |
| **rs4580514** | 3 | intragenic | 2,07E-006 | | **rs3927730** | | CCNA1 | 4,91E-005 |
| **rs11727254** | 4 | intragenic | 6,94E-005 | | **rs753449** | 3 | intragenic | 2,07E-005 |
| **rs2982608** | 11 | ABTB2 | 2,99E-005 | | **rs3893199** | 9 | DAB2iP | 8,97E-005 |
| **rs1455137** | 4 | ANAPC10 | 4,35E-005 | | **rs12490394** | 3 | intragenic | 3,22E-005 |
| **rs11079760** | 17 | CDC27 | 8,07E-006 | | **rs803073** | 7 | CUTL1/SH2B2 | 4,32E-005 |
| **rs721022** | 3 | KPNA4 | 1,24E-005 | | **rs460182** | | RCL1 | 2,74E-005 |
| **rs7617530** | 3 | FHIT | 0,0004321 | | **rs7148162** | 14 | intragenic | 2,07E-005 |
| **rs6897768** | 5 | LOC375449 | 5,10E-005 | | **rs5766175** | 22 | PHF21B | 4,67E-005 |
| **rs2496070** | 1 | VAV3 | 7,53E-005 | | **rs3748483** | 20 | SLC24A3 | 9,29E-005 |
| **rs205965** | 6 | SLC35F1 | 3,93E-005 | | **rs739894** | 22 | OSBP2 | 2,73E-005 |
| | | | | | | | | |
| | | | | | | | | |
| | | | | | | | | |
| | | | | | | | | |
| | | | | | | | | |

In a preferred embodiment of the invention, the method for the diagnosis of an anxiety disorder or a predisposition therefor comprises determining a single polymorphism in a chromosomal copy of at least one of the TMEM132D genes and/or in two chromosomal copies of at least one of said genes, a combination of polymorphisms in a chromosomal copy of at least one of said genes and/or in two chromosomal copies of at least one of said genes and/or a combination of polymorphisms in at least one chromosomal copy of a combination of the FHIT, KPNA4, CRIM1 and PLCB1 genes, wherein said polymorphism and/or combination of polymorphisms is associated with said anxiety disorder or predisposition therefor.

Preferably, the polymorphism of the TMEM132D gene is selected from the group consisting of
(a) the polymorphisms rs7309727, rs900256, rs879560, rs10847832, rs1871899, rs10773613, rs2398469 and rs7979367,
(b) polymorphisms being in linkage disequilibrium with at least one of the polymorphisms of (a), and
(c) combinations of polymorphisms of (a) and/or (b).

The presence of at least one polymorphism associated with anxiety disorder is preferably determined by a genotyping analysis as indicated above. For example, the determination may comprise the analysis of a single polymorphism or of a plurality of polymorphisms in one or two copies of a single gene or in one or two copies of different genes.

The determination may comprise the use of polymorphism-specific primers capable of hybridising with the respective gene and allowing a discrimination between polymorphisms, particularly SNPs at a predetermined position. For example, the genotyping analysis may comprise a primer extension reaction using polymorphism-specific primers as described in the Example. The determination of individual polymorphisms may be carried out as described in the Example.

A further preferred embodiment comprises a microarray analysis which is particularly suitable for the parallel determination of several polymorphisms. Suitable microarray devices are commercially available.

The subjects to be tested are mammals, preferably human beings, suspected to be suffering from anxiety disorder.

As used herein, the term "anxiety disorder" shall mean any pathologic anxiety condition and includes, but is not limited to, different forms of abnormal, pathological anxiety, fears, phobias and nervous conditions that are described as an irrational or illogical worry that is not based on fact. The term anxiety disorder as used herein covers a wide range of severities from general social anxieties to panic disorders, including for example generalized anxiety disorder, panic disorder, agoraphobia, depersonalisation or derealization, phobia, social anxiety disorder, and obsessive compulsive disorder.

The diagnosis of a mental disorder in a human being can be made based on the results of polymorphism/haplotype determination. The patient to be tested may have one or a plurality of polymorphisms and/or at least one combination of polymorphisms of at least one genomic copy which are associated with a mental disorder, preferably schizophrenia. If such a diagnosis is given, the patient is at a higher risk of developing an anxiety disorder.

A further aspect of the disclosure relates to a diagnostic composition or kit for the diagnosis of an anxiety disorder or a predisposition therefor in a mammal, particularly in a human being, comprising at least one primer or probe for determining the presence of at least one polymorphism in the TMEM132D, FHIT, KPNA4, CRIM1, and/or PLCB1 genes, wherein said at least one polymorphism is associated with an anxiety disorder or predisposition therefor.

The primers and/or probes for determining a single polymorphism in a chromosomal copy of said TMEM132D, FHIT, KPNA4, CRIM1, and/or PLCB1 genes and/or in two chromosomal copies of said genes, the combination of primers and/or probes for determining a combination of polymorphisms in a chromosomal copy of said genes and/or in two chromosomal copies of said genes and/or for determining a combination of polymorphisms in at least one chromosomal copy of a combination of the TMEM132D, FHIT, KPNA4, CRIM1, and/or PLCB1 genes, may be nucleic acid molecules such as DNA and RNA or nucleic acid analogues such as peptide nucleic acids (PNA) or locked nucleic acids (LNA). The primers and/or probes are selected such that they can discriminate between polymorphisms at the position to be analysed. Usually, the primers and/or probes have a length of at least 10, preferably at least 15 up to 50, preferably up to 30 nucleic acid building blocks, e.g. nucleotides. In a preferred embodiment, the composition or kit comprises at least one primer and/or probe and/or at least one combination of primers and/or probes which hybridise to the above-mentioned genes under predetermined conditions, e.g. of temperature, buffer, strength and/or concentration of organic solvent, and which allows a specific determination of the polymorphism to be tested.

The composition or kit preferably further comprises an enzyme for primer elongation such as a DNA polymerase, nucleotides, e.g. chain elongation nucleotides, such as desoxy nucleoside triphosphates (dNTPs) or chain termination nucleotides such as didesoxynucleoside triphosphates (ddNTPs) and/or labelling groups, e.g. fluorescent or chromogenic labelling groups.

Still a further aspect of the disclosure relates to a microarray for the diagnosis of an anxiety disorder or a predisposition therefor in a mammal, particularly in a human being, comprising a carrier having immobilised thereto at least one probe for determining the presence of at least one polymorphism and/or of at least one combination of polymorphisms of at least one copy of the TMEM132D, FHIT, KPNA4, CRIM1, and/or PLCB1 genes, wherein said at least one polymorphism is associated with an anxiety disorder or a predisposition therefor.

Preferably, the microarray carrier, e.g. a planar carrier or a microchannel device, has immobilised thereto a plurality of different probes located at different areas on the carrier, which are designed such that they can bind nucleic acid molecules, e.g. RNA molecules or DNA molecules, amplification products, primer elongation products, etc, containing the sequence in which the polymorphism to be tested is located. Thus, an identification of the polymorphism to be analysed by detection of a site-specific binding events of the nucleic acid sample molecule to the probe immobilised on the carrier may be accomplished.

A yet further aspect of the present disclosure is a primer or probe and/or a combination of primers and/or probes for the diagnosis of an anxiety disorder or a predisposition therefor in a mammal, particularly in a human being for determining the presence of at least one polymorphism and/or of at least one combination of polymorphisms of at least one copy of the TMEM132D, FHIT, KPNA4, CRIM1, and/or PLCB1 genes, wherein said at least one polymorphism is associated with an anxiety disorder or a predisposition therefor. The primers may be nucleic acid molecules such as DNA and RNA or nucleic acid analogues such as peptide nucleic acids (PNA) or locked nucleic acids (LNA). The primer and/or probes are selected such that they can discriminate between polymorphisms at the position to be analysed. Usually, the primers and/or probes have a length of at least 10, preferably at least 15 up to 50, preferably up to 30 nucleic acid building blocks, e.g. nucleotides. In a preferred embodiment, the primer hybridises to the above-mentioned genes under predetermined conditions, e.g. of temperature, buffer, strength and/or concentration of organic solvent, and allows a specific determination of the polymorphism to be tested.

Finally, a further aspect of the invention relates to a method of screening for a modulator of an anxiety disorder, comprising determining the effect of a test substance on the expression of at least one copy of the TMEM132D gene, wherein the at least one copy of a gene has at least one polymorphism and/or at least one combination of polymorphisms which is associated with said anxiety disorder or predisposition therefor. Preferably, the at least one polymorphism, the at least one genotype, and/or the at least one combination of polymorphisms is defined as above.

Such methods include cell-based and/or cell-free assays to identify compounds which are capable of interfering with the expression of the TMEM132D gene. According to one aspect of the invention, such method comprises the steps (a) determining the level of expression of the TMEM132D gene in a sample of cells; (b) contacting the sample of cells with a test substance; (c) determining the level of expression of the at least one gene of step (a) for the sample of cells of step (b); and (d) comparing the levels of expression determined in step (a) and (c), wherein an alteration in the level of expression of the at least one gene indicates that the test substance is a modulator of the anxiety disorder.

Furthermore, the present invention shall be explained by the following Figures as well as Examples:

**Figures:**

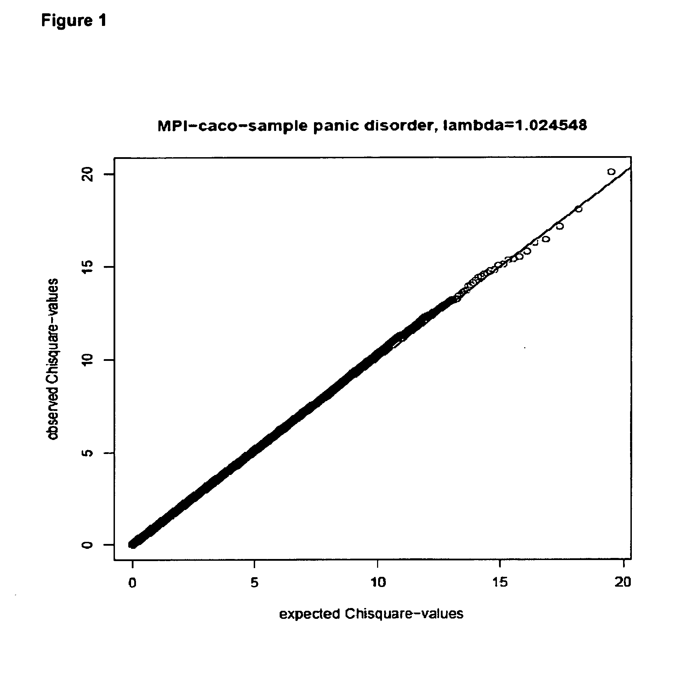

**Figure 1** Extent of population stratification in the MPI sample.
   The distribution of under the null hypothesis expected versus observed _{X}2 values. The p-values were obtained in the analysis of MPI sample, using Armitage trend test with age and sex as covariates, the correction factor is nearly 1 (λ=1.0245). The most values adhere nearly perfect to the diagonal with the expected values without correction for lambda, indicating that possible systematic error due to population stratification even before the correction is small.

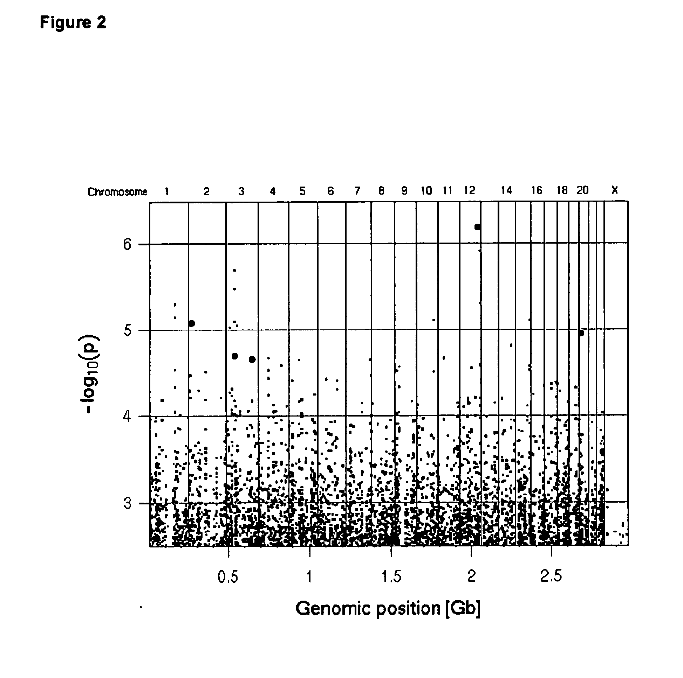

**Figure 2** Genome-wide association study for panic disorder susceptibility loci.
   Results from the first stage analysis using 216 panic cases versus 220 matched controls. The x-axis represents genomic position, the y-axis shows -log¹⁰(p). 5 SNPs with nominal significance in the stage 2 are highlighted in bold.
**Figure 3** Genotype frequencies distribution of rs900256, rs879560 and rs10847832 for the phenotype anticipatory anxiety.

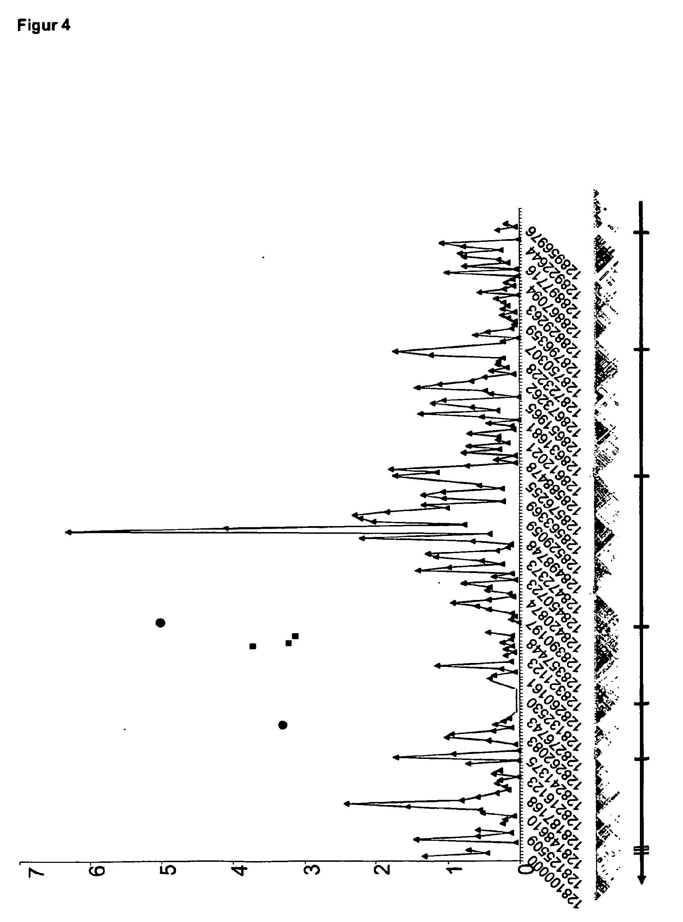

**Figure 4** Summary of the case-control and psychopathology-related results in the gene TMEM132D in the MPI panic sample. P-values are displayed as -log₁₀(Pₙₒₘ): case-control panic: triangles; severity of anticipatory anxiety: squares; Dex-CRH-test: circles.

### METHODS

### Panic sample stage1: Max-Planck Institute (MPI)

222 patients consecutively admitted to the Anxiety Disorders Outpatient Clinic for diagnosis and treatment of panic disorder, mostly presenting with a panic disorder with agoraphobia (83.3%) and panic disorder without agoraphobia (13.7%) as their primary psychiatric diagnoses, were recruited for the study (Table 2).

**Table 2: Demographic data of the genotyped panic samples.**

| | **Sample 1 (MPI** | | | **Sample 2 (Münster)** | | |
|---|---|---|---|---|---|---|
| | cases | controls | p-value | cases | controls | p-value |
| N | 216 | 222 | | 240 | 241 | |
| sex | male 33.2% | male 32.9% | 0.94 | male 40.4% | male 40.2% | 0.97 |
| | female 66.8% | female 67.1% | | female 49.6% | female 49.8% | |
| age (SD) | 38.9 (12.0) | 38.8 (11.7) | 0.58 | 35.8 (10.7) | 38.6 (12.1) | 0.44 |
| diagnosis | PDA 83.3% | none | | PDA 67.5% | none | |
| | PD 16.7% | | | PD 32.5% | | |

| | | | | | | |
|---|---|---|---|---|---|---|
| PDA: Panic disorder with agoraphobia; PD: Panic disorder without agoraphobia; SD: standard deviation. | | | | | | |

The diagnosis was ascertained by trained psychiatrists according to the Diagnostic and Statistical Manual of Mental Disorders (DSM)-IV criteria. All patients underwent the Structured Clinical Interviews for DSM-IV (SKID I and II). Panic disorder due to a medical or neurological condition or a comorbid Axis II disorder were exclusion criteria. All patients underwent a thorough clinical examination including EEG, ECG and detailed hormone laboratory assessment. The mean age at onset of the disorder was 27.4 (SD: 11.6). The Panic and Agoraphobia scale (Bandelow 1995) indicated a moderate severity of panic and agoraphobia (mean score (SD) rating assessed by clinicians: 29.8 (9.8)). The mean Hamilton Depression Scale score was 13.9 (6.7) and mean Hamilton Anxiety Scale score was 23.9 (9.9), indicating low depression and moderate anxiety in the patients (Hamilton 1959; 1960).

Ethnicity was recorded using a self-report sheet for perceived nationality, mother language and ethnicity of the subject itself and all 4 grandparents. All included patients were Caucasian: 83.0% of German origin, 17.0% of European origin, excluding Turkish origin. The study was approved by the local ethics committee. Written informed consent was obtained from all subjects.

222 controls matched for ethnicity (using the same questionnaire as for patients), gender and age were recruited (demographic data in Table 2). Controls were selected randomly from a Munich-based community samples and screened for the presence of anxiety and affective disorders using the Munich version of the Composite International Diagnostic-Screener (M-CIDI) (lit). The M-CIDI is an updated version of the World Health Organization's CIDI version 1.2 (WHO-CIDI; World Health Organization, 1992), which incorporates questions to cover DSM-IV (American Psychiatric Association, 1994) and ICD-10 (World Health Organization, 1990) diagnostic criteria. Only individuals negative in the screening questions for the above-named disorders were included in the sample. These subjects thus represent a group of individuals from the general population who have never been mentally ill. Recruitment of controls was also approved by the local ethics committee and written informed consent was obtained from all subjects.

### Panic sample stage2: Münster

240 patients with panic disorder with or without agoraphobia as their primary diagnoses as well as 241 anonymous blood donor controls matched for ethnicity, gender and age were used as a replication sample (see Table 2). The diagnosis was ascertained by trained psychiatrists according to the Diagnostic and Statistical Manual of Mental Disorders (DSM)-IIIR criteria in 37.5% of patients and according to DSM-IV in 62.5% of patients on the basis of structured interviews (SADS-LA and CIDI) and clinical records as previously described (Sang et al., 2002). Recruitment of subjects was approved by the local ethics committee and written informed consent was obtained from all subjects.

### DNA preparation

On enrolment in the study, up to 40 ml of EDTA blood were drawn from each patient and DNA was extracted from fresh blood using standard DNA extraction procedures, e.g. the Puregene whole blood DNA-extraction kit (Gentra Systems Inc; MN)(Binder et al. 2004).

### SNP Genotyping

Genome-wide SNP genotyping for all patients from the MPI sample was performed on Sentrix Human-1 Genotyping BeadChips (Illumina Inc., San Diego, USA) according to the manufacturer's standard protocols. On the Illumina Human-1 Genotyping BeadChips more than 370,000 SNPs can be interrogated, which have been selected from the entire genome, mainly based on HapMap tagging SNPs and to a minor part with an exon-centric focus. The average call rate achieved was better than 99%, with samples below 98% being either retyped or excluded from the study. The reproducibility for samples (N = 3) genotyped twice was 99.999% or better.

The genotyping in the second stage (Münster sample) was performed on a MALDI-TOF mass-spectrometer (MassArray® system) employing the AssayDesigner software (Sequenom Inc., San Diego, USA) for primer selection, multiplexing and assay design, and the homogeneous mass-extension (hMe) process for producing primer extension products. MALDI-TOF SNP genotyping was performed at the facilities of the Institute of Human Genetics, GSF - National Research Center for Environment and Health, Neuherberg, Germany. All oligonucleotide sequences used are available upon request.

### Statistics

Genome-wide case-control analyses were conducted by applying the WG-Permer software (http://wg-permer.org) and R-2.5.0 (http://cran.r-project.org). SNPs deviating from Hardy-Weinberg-equilibrium as well as SNPs with a callrate below 98% (initial sample) respectively 90% (stage 2 sample). The difference being owed to the two different genotyping methods used. SNPs with a minor allele frequency below 2.5% were not taken into further analysis. SNPs were tested for association with panic disorders using the allelic _{X}²-test. Results of the significant SNPs were also verified with the Armitage test for trend. The level of significance was set to 5% (family-wise error rate, FER), and p-values were corrected for multiple comparisons by Bonferroni method. Power calculations were performed using CaTS (http://www.sph.umich.edu/csg/abecasis/CaTS). Population stratification was tested with EIGENSTRAT implemented in EIGENSOFT (http://genepath.med.havard.edu/-reich/EIGENSTRAT.htm).

Analyses for psychopathology-related phenotypes (e.g. severity of panic attacks) were performed using generalized linear method with SPSS for Windows (Releases 12, SPSS Inc., Chicago, Illinois 60606, USA) testing effects on the genotypic level. Age and gender were used as covariates for all analyses. For the analysis of the linkage disequilibrium (LD) pattern and haplotype block delineation we used haplotypes estimated by Haploview (http://www.broad.mit.edu/personal/jcbarret/haplo/). Blocks were defined using the confidence interval method described by Gabriel (Gabriel et al., 2002). For the analysis of haplotypic association we used COCAPHASE (Figure 1).

### RESULTS

Using Illumina Sentrix Human-1 300k BeadChip, around 317,503 autosomal SNPs were genotyped. To minimize the risk of false positive results all SNPs with a call rate of less than 98% (9,988 SNPs), or/and displaying deviation from Hardy-Weinberg equilibrium (HWE) at an error level of below 10⁻⁵ (8,795 SNPs) were excluded. As rare polymorphisms have low power to show a significant effect, also all SNPs with a minor allele frequency (MAF) below 2.5% (1,058 SNPs) were excluded from the analysis. This resulted in 297,979 SNPs that were considered for data analysis. 15,472 (5.2%) among all analyzed SNPs showed a nominally significant deviation from HWE with the level of significance set to 5%, which is almost identical to the expected number of false positive findings under the null hypothesis of no HWE deviations. The average case-wise call rate across all SNPs was 99.8%.

To test for the possible effects of population stratification, the method of genomic controls was applied. P-values were calculated for the allelic _{X}² - test for all SNPs passing the quality control criteria mentioned above. The distribution of these p-values was compared to the theoretical expected _{X}² - distribution. λ the quotient between the median of the test statistics and the median of the theoretical distribution is a measure for stratification. This quotient is 1.025 in the initial sample which implies that no large effects of population stratification are present. Furthermore, EIGENSTRAT was ran, a method based on principal component analysis, which detects and corrects for population stratification. No outliers were detected which is another indicator that population stratification seems not to be problematic in our data.

The highest nominal case-control association in the whole genome genotyping was found for rs7309727 (allelic test: p=5.1 e-07, Armitage test for trend: p=7.726e-07) located in the gene TMEM132D (see Table 3 for genotypes). None of the nominal genome-wide associations withstood the correction for multiple testing using Bonferroni.

**Table 3: Genotype frequency distribution and case-control p-values for rs7309727 using Armitage's trend test in the MPI sample, Münster sample and in the joint sample.**

| | **Sample 1 (MPI)** | | | **Sample 2 (Münster)** | | | **Joint Sample** |
|---|---|---|---|---|---|---|---|
| | controls | cases | p-value | controls | cases | p-value | p-value |
| **rs7309727** | | | | | | | |
| HWE | 0.29 | 0.28 | p=7.7e-07 | 0.44 | 0.42 | p=006 | p=6.9e-08 |
| CC (n / %) | 154 / 69.4% | 100 / 46.3% | OR=2.1 | 123 / 60.6% | 111 / 50.5% | OR=1.5 | corrected: 0.021 |
| CT (n / %) | 59 / 26.6% | 94 / 43.5% | CI=1.58-3.02 | | 87 / 39.5% | CI=1.11-2.07 | corrected^{λ} 0.03 |
| TT (n / %) | 9/4.1% | 22 / 10.2% | | 8 / 3.8% | 22 / 10.0% | | OR=1.8 |
| | | | | | | | CI=1.46-2.28 |

Therefore, in the second stage, 64 SNPs were selected with the highest nominal p-values ranging up to p=1.0e-04 from the first stage and they were analyzed in independent panic sample from Münster. 56 SNPs passed the quality criteria in the second stage (call rate > 90%, HETEX > 10, MAF > 2.5%). 5 SNPs from the stage 2 showed a nominal case-control association either with panic disorder (4 SNPs) or with agoraphobia (1 SNP). The highest associated SNP from stage 1 (rs7309727/TMEM132D) displayed a nominal case-control association with panic disorder in the stage 2 (allelic test p=0.008, Armitage test 0.008, see Table 3). The allele T was found as risk allele for panic disorder in both samples.

Further nominal associations were found for rs7617530 (FHIT gene; MPI sample: p=0.0009; Münster sample: p=0.007), rs721022 (KPNA4 gene; MPI sample: p=0.00001; Münster sample: p=0.007) and rs12466450 (CRIM1 gene; MPI sample: p=0.0001; Münster sample: p=0.008). rs8118127, located in the PLCB1 gene and originally associated with panic disorder in the MPI sample (p=9.0e-05), showed a nominal case-control association with the phenotype PDA in the Münster sample (p=0.001).

In order to increase the power to detect true positive findings further the statistical analysis was performed in the joint sample. For rs7309727, the significance of the association with panic disorder in the joint sample increased, the obtained p-value (p=6.9e-8) withstood the genome-wide correction for multiple testing (corrected p=0.021; see specifics in Table 3). For rs7617530, the p-value decreased to p=1.0e-5 (OR=1.5; CI: 1.2-1.9), but withstood not the correction procedure. For the remaining SNPs, the p-values increased in the joint sample (rs12466450: p=0.003; rs721022: p=0.008; rs8118127: p=0.003).

The power to detect a true genome-wide significant association of the type found for rs7309727 was calculated. For the MPI sample, the power ranges to 75% with an attributable risk of 42.8. In the Münster sample the power was 57% and the power in the joint sample was 62% with an attributable risk of 38.1%.

### Additional evidence

To further evaluate the putative role of the gene TMEM132D in panic disorder and psychopathology, a calculation was performed using quantitative phenotypes in the MPI panic sample. In the sample used in stage 2 only case-control phenotypes were available.

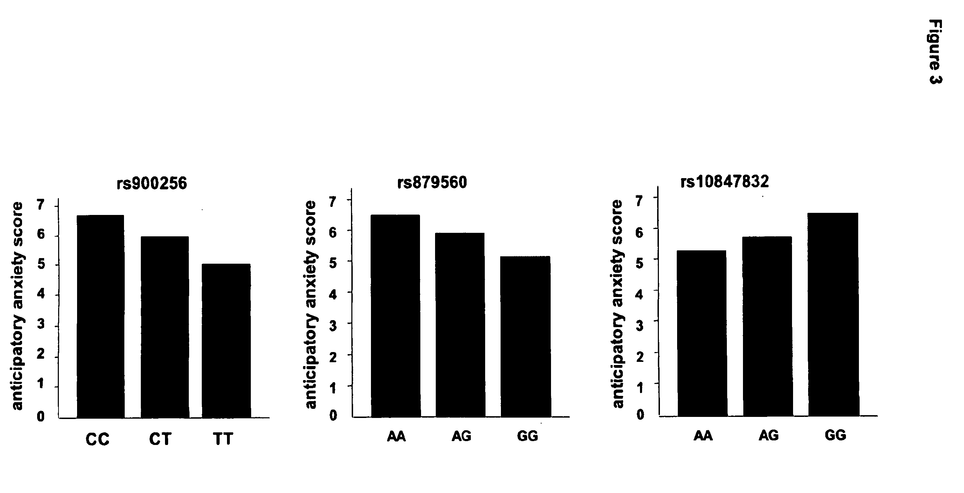

TMEM132D gene spans over 830kb on Chromosome 12q24.32 - 12q24.33. All SNPs in the gene region ± 20kb were included for the further analysis, this region was covered with 172 SNPs on 300k Illumina Chip, 170 SNPs turned to be polymorphic. All 170 polymorphic SNPs were tested for association with disease severity using "The Panic and Agoraphobia scale" total score for a rating reflecting the worst episode of panic symptoms (PA: obtained by clinicians), the psychopathology values were corrected for age and gender before the analysis. When the total score was significant or showed a trend for association, subscales were tested for significant effects to further specify the phenotypic effects. The highest association with the total score in the Panic- and Agoraphobia Scale was found for rs900256 (p=0.0003). Further testing pointed towards effects in severity of anticipatory anxiety (p=0.0001) and moderate effect for agoraphobia (p=0.001) for rs900256 with highest severity values in the CC allele carrier. Regarding the subscales, the associations were found mainly for anticipatory anxiety, the highest p-values were obtained for rs879560 (p=0.0006, risk allele A) and rs10847832 (p=0.0007, risk allele A) (see for genotypes Figure 3). After correction for multiple testing 170 analyzed SNPs, the association for rs900256 with anticipatory anxiety remained significant (after correction p=0.03).

Further, an association was found for rs1871899 (p=0.0001) and rs10773613 (p=0.0006) with the parameter age at onset; the association with rs1871899 remained significant after correction for multiple testing (p=0.02). There were no association with the Hamilton anxiety or Hamilton Depression Score after correction for multiple testing in the MPI panic sample.

### DISCUSSION

This first genome-wide association study has detected an association in the gene TMEM132D, further loci have been found, but were of lower statistical evidence. In the TMEM132D gene, one SNP was strongly associated with panic disorder in the initial sample and displayed nominal significance in an independent German panic sample. In the joint sample, the significance of association increased and withstood the genome-wide correction for multiple testing. Further, the role of the genetic variances in the TMEM132D was evaluated for the severity of panic attacks and an association with rs900256 with the anticipatory anxiety was found. For this phenotype, further two SNPs showed a nominal association in the TMEM132D. This finding is very interesting, because the expression analysis for TMEM132D show strong expression in the cortical areas, hippocampus and amygdala, where anxiety-related stimuli and behavioural reactions are supposed to be processed and generated. Interestingly, in a previous linkage study with panic and agoraphobia patients performed by Smoller et al. (2001), linkage to the chromosomal region 12q13.13 was found. Although this is not directly the region of the present gene (chr 12q31), linkage peaks could be related to a large genomic region.

In summary, a genome-wide study in panic disorder was performed and revealed a gene, that is strongly associated on different levels, case-control, psychopathology and endocrine level. These are very promising results and the gene have to be validated now on functional level. All associated SNPs were located in intronic regions which could have regulatory functions. This first genome-wide association study of panic disorder has important implications for future genetic and biological studies for this common and disabling brain disease.

## Claims

1. A method for the diagnosis of an anxiety disorder or a predisposition therefor in a mammal, particularly in a human being, comprising determining the presence of at least one polymorphism of the TMEM132D gene, wherein the polymorphism is selected from the group consisting of rs7309727, rs900256, rs879560, rs10847832, rs1871899, rs10773613, rs2398469 and rs7979367.

2. The method of claim 1 comprising:
(a) determining a single polymorphism in a chromosomal copy of the gene, wherein said polymorphism is associated with said anxiety disorder or predisposition therefor;
(b) determining a single polymorphism in two chromosomal copies of the gene, wherein said polymorphism is associated with said anxiety disorder or predisposition therefor;
(c) determining a combination of polymorphisms in a chromosomal copy of the gene, wherein said combination of polymorphisms is associated with said anxiety disorder or predisposition therefor;
(d) determining a combination of polymorphisms in two chromosomal copies of the gene, wherein said combination of polymorphisms is associated with said mental disorder or predisposition therefor;
(e) determining a combination of polymorphisms in at least one chromosomal copy of a combination of genes, wherein said combination of polymorphisms is associated with said mental disorder or predisposition therefor.

3. The method of claim 1 or 2, wherein a combination of polymorphisms in at least one chromosomal copy of the TMEM132D gene is determined.

4. The method of any one of claims 1 to 3, wherein the polymorphism is determined by a genotyping analysis.

5. The method of claim 4, wherein the genotyping analysis comprises the use of polymorphism-specific primers, a mass-spectrometric analysis, and/or a microarray analysis.

6. An in vitro method of screening for a modulator of an anxiety disorder, comprising determining the effect of a test substance on the level of expression of at least one copy of the TMEM132D gene, wherein the at least one copy of a gene has at least one polymorphism, and/or at least one combination of polymorphisms which is associated with said anxiety disorder or predisposition therefor, wherein the at least one polymorphism is defined as in claim 1, and/or a combination of said polymorphisms.

## Patentansprüche

1. Verfahren zur Diagnose einer Angststörung oder einer Prädisposition dafür in einem Säugetier, insbesondere in einem Menschen, umfassend Bestimmen der Anwesenheit mindestens eines Polymporphismus des TMEM132D-Gens, wobei der Polymorphismus ausgewählt ist aus der Gruppe bestehend aus rs7309727, rs900256, rs879560, rs10847832, rs1871899, rs10773613, rs2398469 und rs7979367.

2. Verfahren nach Anspruch 1 umfassend:
(a) Bestimmen eines einzelnen Polymorphismus in einer Chromosomenkopie des Gens, wobei der Polymorphismus mit der Angststörung oder Prädisposition dafür assoziiert ist;
(b) Bestimmen eines einzelnen Polymorphismus in zwei Chromosomenkopien des Gens, wobei der Polymorphismus mit der Angststörung oder Prädisposition dafür assoziiert ist;
(c) Bestimmen einer Kombination an Polymorphismen in einer Chromosomenkopie des Gens, wobei die Kombination an Polymorphismen mit der Angststörung oder Prädisposition dafür assoziiert ist;
(d) Bestimmen einer Kombination an Polymorphismen in zwei Chromosomenkopien des Gens, wobei die Kombination an Polymorphismen mit der psychischen Störung oder Prädisposition dafür assoziiert ist;
(e) Bestimmen einer Kombination an Polymorphismen in mindestens einer Chromosomenkopie einer Kombination an Genen, wobei die Kombination an Polymorphismen mit der psychischen Störung oder Prädisposition dafür assoziiert ist.

3. Verfahren nach Anspruch 1 oder 2, wobei eine Kombination an Polymorphismen in mindestens einer Chromosomenkopie des TMEM132D-Gens bestimmt wird.

4. Verfahren nach einem der Ansprüche 1 bis 3, wobei der Polymorphismus durch eine Genotypisierungs-Analyse bestimmt wird.

5. Verfahren nach Anspruch 4, wobei die Genotypisierungs-Analyse die Verwendung von Polymorphismus-spezifischen Primern, einer massenspektrometrischen Analyse und/oder einer Microarray-Analyse umfasst.

6. In vitro Verfahren zum Screening eines Modulators einer Angststörung, umfassend Bestimmen des Effekts einer Testsubstanz auf das Expressionslevel mindestens einer Kopie des TMEM132D-Gens, wobei die mindestens eine Kopie eines Gens mindestens einen Polymorphismus und/oder mindestens eine Kombination an Polymorphismen aufweist, der/die mit der Angststörung oder Prädisposition dafür assoziiert ist, wobei der
mindestens eine Polymorphismus wie in Anspruch 1 definiert ist und/oder eine Kombination der Polymorphismen.

## Revendications

1. Procédé pour le diagnostic d'un désordre de l'anxiété ou d'une prédisposition à celui-ci chez un mammifère, en particulier un être humain, comprenant la détermination de la présence d'au moins un polymorphisme du gène TMEM132D, où le polymorphisme est choisi parmi le groupe consistant en rs7309727, rs900256, rs879560, rs10847832, rs1871899, rs10773613, rs2398469 et rs7979367.

2. Procédé selon la revendication 1, comprenant :
(a) la détermination d'un seul polymorphisme dans une copie chromosomique du gène, où ledit polymorphisme est associé audit désordre de l'anxiété ou à sa prédisposition ;
(b) la détermination d'un seul polymorphisme dans deux copies chromosomiques du gène, où ledit polymorphisme est associé audit désordre de l'anxiété ou à sa prédisposition ;
(c) la détermination d'une combinaison de polymorphismes dans une copie chromosomique du gène, où ladite combinaison de polymorphismes est associée audit désordre de l'anxiété ou à sa prédisposition ;
(d) la détermination d'une combinaison de polymorphismes dans deux copies chromosomiques du gène, où ladite combinaison de polymorphismes est associée audit désordre mental ou à sa prédisposition ;
(e) la détermination d'une combinaison de polymorphismes dans au moins une copie chromosomique d'une combinaison de gènes, où ladite combinaison de polymorphismes est associée audit désordre mental ou à sa prédisposition.

3. Procédé selon la revendication 1 ou 2, où une combinaison de polymorphismes dans au moins une copie chromosomique du gène TMEM132D est déterminée.

4. Procédé selon l'une quelconque des revendications 1 à 3, où le polymorphisme est déterminé par une analyse génotypique.

5. Procédé selon la revendication 4, où l'analyse génotypique comprend l'utilisation d'amorces spécifiques de polymorphisme, une analyse par spectrométrie de masse et/ou une analyse par puce.

6. Procédé *in vitro* de criblage d'un modulateur d'un désordre de l'anxiété, comprenant la détermination de l'effet d'une substance test sur le taux d'expression d'au moins une copie du gène TMEM132D, où la au moins une copie du gène a au moins un polymorphisme, et/ou au moins une combinaison de polymorphismes, qui est associée audit désordre de l'anxiété ou à sa prédisposition, où le au moins un polymorphisme est défini à la revendication 1, et/ou une combinaison desdits polymorphismes.
